# EUROPÄISCHE PATENTSCHRIFT

(11) **EP 3 714 759 B1**
(45) Veröffentlichungstag und Bekanntmachung des Hinweises auf die Patenterteilung: **18.01.2023**
(21) Anmeldenummer: 20161104.3
(22) Anmeldetag: 05.03.2020
(51) Int. Cl.: A61B 1/00

(54) **VERFAHREN ZUM HERSTELLEN EINES ENDOSKOPS**
METHOD FOR PRODUCING AN ENDOSCOPE
PROCÉDÉ DE FABRICATION D'UN ENDOSCOPE

(30) Priorität: 28.03.2019 DE 102019108117
(43) Veröffentlichungstag der Anmeldung: 30.09.2020
(73) Patentinhaber: OLYMPUS Winter & Ibe GmbH, 22045 Hamburg (DE)
(72) Erfinder: SCHÖLER, Uwe, 22955 Hoisdorf (DE); TORKUHL, Nils, 22888 Barsbüttel (DE)
(74) Vertreter: Seemann & Partner Patentanwälte mbB

(56) Entgegenhaltungen:
- DE-A1-102014 215 793
- JP-A- 2008 184 691
- US-A- 5 944 656
- US-A1- 2014 330 082

## Beschreibung

Die Erfindung betrifft ein Verfahren zum Herstellen eines Endoskops sowie die Verwendung eines Fensters und einer Löt-Preform zur Herstellung eines einen Endoskopschaft aufweisenden Endoskops.

Video-Endoskope weisen üblicherweise an deren proximalen Enden ein Fenster auf, um Gegenstände, die am distalen Ende oder in der Nähe des distalen Endes des Endoskops zu beobachten sind, betrachten zu können. Im Inneren des Video-Endoskops im Bereich des Fensters, d.h. am proximalen Ende kann beispielsweise eine Kamera vorgesehen sein, die durch ein entsprechendes Fenster, das am distalen Ende des Endoskops vorgesehen ist, Bilder aufnehmen kann. Alternativ können auch Lichtleitfasern und gegebenenfalls eine Optik vorgesehen sein, um Bilder vom distalen Ende des Endoskops zum proximalen Ende zu leiten.

Bei Endoskopen, die in der Medizintechnik verwendet werden, ist es notwendig, dass die Endoskope ausreichend gereinigt und sterilisiert werden können. Hierzu müssen diese autoklavierbar, spülmaschinenfest und auch geeignet sein, in einer aggressiven chemischen Umgebung stabil zu sein. Ein in einem z.B. starren Endoskop eingebrachtes Fenster muss somit hermetisch dichtend eingebracht sein. Hierzu ist es bekannt, die Seitenwände des Fensters, die nach Einbau des Fensters in das Rohr an das Rohrmaterial angrenzen, in einem mehrschichtigen Prozess zu metallisieren, um diese dann anschließend zu verlöten. Dieses ist relativ aufwendig. Zudem ist es erforderlich, ein Lot zu verwenden, das insbesondere bei Verwendung von ätzenden Chemikalien zur Reinigung des Endoskops nicht angegriffen wird.

Darüber hinaus befindet sich am proximalen Ende des Endoskops beispielsweise ein Okulartrichter mit einem Okular, also einer optischen Baugruppe, aus der das an der distalen Spitze des Endoskops eingetretene Licht wieder austritt. Ein solches im hermetischen Raum vorgesehenes Okular kann zur direkten Beobachtung des Operationsfeldes mit bloßem Auge verwendet werden. Vielfach ist vorgesehen, dass an das Okular ein Kamerakopf angeschlossen ist, so dass das Operationsfeld auf einem Monitor betrachtet oder die erfassten Bilddaten einer sich anschließenden Bildverarbeitung zugeführt werden können. Ein solches Endoskop geht beispielsweise aus EP 0 501 088 A1 hervor.

Die in einem Innenraum des Endoskops, beispielsweise in dessen Schaft, vorhandenen optischen Baugruppen sind gegenüber einem Außenraum, der das Endoskop umgibt, durch ein Okularfenster voneinander getrennt.

Bei starren Endoskopen oder Videoskopen werden die optischen Systeme am distalen und am proximalen Ende hermetisch jeweils mit einer Saphirscheibe abgeschlossen, die in eine distalseitige Faserrohrspitze bzw. in eine proximalseitige Okularfensterfassung eingefügt sind. Distalseitig erfolgt der Fügeprozess durch manuelles Löten, während proximalseitig die Saphirscheibe in die Okularfensterfassung geklebt und mit einer Überwurfmutter gesichert wird.

US 2014/330082 A1 offenbart ein Endoskop, wobei an dem proximalen Ende eines Objektivkopfs ein Fenster mit einer kappenartigen Metallabdeckung vorgesehen ist.

Ferner ist in US 5,944,656 A1 ein Endoskop offenbart, wobei in einem Schaft mehrere Linsen durch zylindrische Abstandshalter in Position gehalten werden.

Darüber hinaus ist in JP 2008-184691 A ein Sichtfenster eines Vakuumbehälters beschrieben.

Außerdem beschreibt DE 10 2014 215 793 A1 ein Verfahren zum Herstellen einer Verbindung zwischen zwei Bauteilen, wobei ein Verbindungsformteil aus einem thermoplastischen Material bereitgestellt wird, das zwischen einem ersten Bauteil und einem zweiten Bauteil angeordnet wird.

Es ist Aufgabe der vorliegenden Erfindung, ein einfaches und sicheres Einbringen eines Fensters in ein, vorzugsweise distales oder proximales, Ende des Endoskops zu ermöglichen, wodurch sich ein autoklavierbares sowie chemisch stabiles Endoskop herstellen lässt.

Die Erfindung ist in den Ansprüchen 1 und 10 definiert.

Gelöst wird obige Aufgabe durch ein Verfahren zum Herstellen eines Endoskops, mit den folgenden Verfahrensschritten:
- Bereitstellen eines eine seitliche Umfangsfläche und zwei Planflächen aufweisenden Fensters, wobei das Fenster an der seitlichen Umfangsfläche und/oder im Randbereich einer Planfläche des Fensters mit einer metallischen Beschichtung versehen ist, und wobei eine oder die, vorzugweise mit der metallischen Beschichtung versehene, Planfläche des Fensters mit einer Löt-Preform versehen ist oder wird,
- Einsetzen des mit der Löt-Preform versehenen Fensters in eine Fensteraufnahme eines Endoskopschafts des Endoskops, wobei insbesondere die mit der Löt-Preform versehene Planfläche des Fensters dem Innenraum des Endoskopschafts zugewandt oder vom Innenraum des Endoskopschafts abgewandt ist,
- Erwärmen, vorzugsweise gleichzeitiges Erwärmen, der Löt-Preform an mehreren Stellen oder, vorzugsweise gleichzeitiges, Erwärmen der gesamten Löt-Preform mittels einer Heizvorrichtung, so dass das fließfähige Lotmaterial der Löt-Preform zwischen dem Fenster und der Fensteraufnahme verteilt wird und nach Abkühlung des Lotmaterials ein Lot zwischen dem Fenster und der Fensteraufnahme angeordnet ist.

Die Erfindung beruht auf dem Gedanken, dass durch die Bereitstellung eines Löt-Preforms, die zwischen der Planfläche des in die Fensterfassung eingesetzten Fensters und dem Innenraum des Endoskopschafts oder zwischen der Planfläche des Fensters und dem Außenraum des Endoskops angeordnet ist, eine einfache Verlötung des Fensters mit der Fensteraufnahme erreicht wird. Hierbei wird die Löt-Preform beispielsweise mittels einer induktiven Heizeinrichtung oder eines Lasers teilweise oder komplett erwärmt, wodurch das Lotmaterial der Löt-Preform fließfähig wird und dadurch in den Zwischenraum zwischen dem Fenster bzw. der eingesetzten Fensterscheibe und der Innenwandung sowie der Auflagefläche der Fensteraufnahme für das Fenster fließt, wodurch eine hermetische und chemisch stabile Lotschicht zwischen dem Fenster und der Fensteraufnahme ausgebildet ist.

Insbesondere sind die seitliche Umfangsfläche des Fensters und die Planfläche des Fensters jeweils mit einer metallischen Beschichtung in einer Ausgestaltung versehen. Außerdem sind die der seitlichen Umfangsfläche des Fensters zugewandte Innenwandung der Fensteraufnahme und/oder die der Planfläche des Fensters zugewandte Auflagefläche der Fensteraufnahme ebenfalls mit einer metallischen Beschichtung versehen. Vorzugsweise weisen die metallischen Beschichtungen für die Umfangsfläche und/oder die Planfläche des Fensters und/oder die metallischen Beschichtungen für die Innenwandung und die Auflagefläche der Fensteraufnahme jeweils Gold auf oder sind aus Gold hergestellt.

Die auf der Planfläche des Fensters aufgebrachte metallische Beschichtung ist bevorzugter Weise ringförmig und am Rand des Fensters ausgebildet, wodurch die metallische Beschichtung auf der Planfläche einen inneren Bereich für einen Lichtdurchtritt durch das Fenster umgibt.

Die Fixierung des Fensters in der Fensteraufnahme bzw. Fensterfassung erfolgt hierbei ohne Klebemittel, d. h. klebemittelfrei, wodurch die Verlötung des Fensters in der Fensteraufnahme vereinfacht wird. Vorzugsweise wird ein eine seitliche Umfangsfläche und zwei parallele Planflächen aufweisendes Fenster bereitgestellt, wobei das Fenster an der seitlichen Umfangsfläche und/oder im Randbereich ausschließlich einer Planfläche des Fensters mit einer metallischen Beschichtung versehen ist, und wobei auf der, vorzugweise mit der metallischen Beschichtung versehenen, Planfläche des Fensters eine Löt-Preform aufgebracht ist oder wird. Das Fenster wird in eine Fensteraufnahme eines Endoskopschafts des Endoskops eingesetzt, wobei die, vorzugweise mit der metallischen Beschichtung versehene, Planfläche des Fensters und die Löt-Preform dem Innenraum des Endoskopschafts zugewandt oder vom Innenraum des Endoskopschafts abgewandt sind.

Es ist vorgesehen, dass hierdurch beispielsweise ein, vorzugsweise starres, Endoskop mit einem Linsensystem oder ein Video-Endoskop bzw. Videoskop hergestellt ist, wobei wenigstens ein Fenster in einer Fensteraufnahme des Endoskops oder des Video-Endoskops bzw. Videoskops verlötet ist.

Es ist hierbei vorgesehen, dass mittels der Verfahrensschritte ein Fenster bzw. eine Fensterscheibe beispielsweise an einer distalen Faserrohrspitze oder an einer proximalen Okularfassung verlötet wird.

Durch die komplette und gleichzeitige Erwärmung der Löt-Preform wird bewirkt, dass nach dem Erkalten des Lotmaterials eine Selbstzentrierung des Fensters innerhalb der Fensteraufnahme erreicht wird.

Darüber hinaus besteht ein weiterer Vorteil des Verfahrens darin, dass eine lunkenfreie bzw. nahezu lunkenfreie Verlötung im Zwischenraum zwischen der Fensteraufnahme und dem Fenster erreicht wird.

Gemäß einem bevorzugten Verfahrensschritt ist weiter vorgesehen, dass die metallische Beschichtung des Fensters aus Gold besteht oder eine äußere Schicht Gold aufweist.

Des Weiteren ist es bei dem Verfahren bevorzugt, dass die Fensteraufnahme Flächen, die der seitlichen Umfangsfläche des Fensters und der Planfläche des Fensters zugewandt sind, aufweist, wobei wenigstens eine Fläche oder beide Flächen eine Goldbeschichtung oder eine äußere Goldschicht aufweisen. Hierbei sind die Flächen der Fensteraufnahme eine umlaufende Innenwandung, die der seitlichen Umfangsfläche des Fensters zugewandt ist, und eine Auflagefläche für das Fenster, die der Planfläche des Fensters zugewandt ist. In einer Ausführungsform ist hierbei vorgesehen, dass zwischen der mit einer metallischen Beschichtung versehenen Planfläche des Fensters am Rand des Fensters und der ringförmigen Auflagefläche der Fensteraufnahme für die Planfläche des Fensters die Löt-Preform angeordnet ist.

Dazu ist gemäß einem bevorzugten Aspekt vorgesehen, dass die Löt-Preform ringförmig oder scheibenförmig ausgebildet ist.

Gemäß einem weiteren Aspekt ist vorzugsweise die Löt-Preform aus Gold und Zinn gebildet. Beispielsweise weist die Löt-Preform eine Mischung aus 80 % Gold und 20 % Zinn auf.

Um die Löt-Preform zu erwärmen, ist hierbei in einer Ausgestaltung die Heizvorrichtung als induktive Heizvorrichtung oder als Lasereinrichtung ausgebildet. Mittels der induktiven Heizvorrichtung oder der Lasereinrichtung, zum Beispiel ein Dioden-Laser, wird die Löt-Preform gleichzeitig und komplett erwärmt, wodurch das Fenster in der hierfür vorgesehenen Fensteraufnahme des Endoskopschafts verlötet wird.

Bevorzugterweise ist das Fenster zylinderförmig und/oder als, vorzugsweise scheibenförmiges, Planglas ausgebildet.

Insbesondere ist gemäß einem weiteren Aspekt das Fenster aus Saphirglas hergestellt.

Gemäß einer bevorzugten Weiterbildung ist vorgesehen, dass die Fensteraufnahme aus Stahl oder aus Kunststoff, insbesondere PEEK (Polyetheretherketon), hergestellt ist. Das Fenster kann somit in eine Fensteraufnahme aus Stahl, zum Beispiel an einer Faserrohrspitze eingesetzt werden. In einer anderen Ausgestaltung ist es darüber hinaus möglich, das Fenster in einer aus Kunststoff bestehenden Okularfassung am proximalen Ende des Endoskopschafts zu verlöten.

Bevorzugterweise ist die Fensteraufnahme am distalen Ende des Endoskopschafts, z.B. an einer distalen Faserrohrspitze eines Faserrohrs, oder am proximalen Ende des Endoskopschafts, z.B. in einer Fensteraufnahme einer Okulareinrichtung bzw. eines Okulars, ausgebildet.

Insbesondere ist die metallische Beschichtung auf der Planfläche des Fensters ringförmig ausgebildet ist und/oder weist eine Ringbreite zwischen 50 µm und 1000 µm, insbesondere zwischen 100 µm und 500 µm, auf.

Ferner wird die Aufgabe gelöst durch eine Verwendung eines Fensters und einer Löt-Preform zur Herstellung eines einen Endoskopschaft aufweisenden Endoskops, vorzugweise starres Endoskop oder Video-Endoskop, wobei das Fenster eine seitliche Umfangsfläche und zwei Planflächen aufweist, wobei das Fenster an der seitlichen Umfangsfläche und/oder im Randbereich einer Planfläche des Fensters mit einer metallischen Beschichtung versehen ist, und wobei eine oder die, vorzugweise mit der metallischen Beschichtung versehene, Planfläche des Fensters mit einer Löt-Preform versehen ist oder wird, wobei das Fenster in eine Fensteraufnahme des Endoskopschafts einsetzbar ist oder eingesetzt wird, wobei die mit der Löt-Preform versehene Planfläche des Fensters dem Innenraum des Endoskopschafts zugewandt oder vom Innenraum des Endoskopschafts abgewandt ist.

Dabei ist es in einer Weiterbildung bevorzugt, dass durch gleichzeitiges Erwärmen der Löt-Preform an mehreren Stellen oder durch gleichzeitiges Erwärmen der gesamten Löt-Preform mittels einer Heizvorrichtung das fließfähige Lotmaterial der Löt-Preform zwischen dem Fenster und der Fensteraufnahme verteilbar oder verteilt ist und nach Abkühlung des Lotmaterials ein Lot zwischen dem Fenster und der Fensteraufnahme angeordnet ist.

Ferner ist gemäß einer Ausgestaltung vorgesehen, dass die metallische Beschichtung des Fensters aus Gold besteht oder eine äußere Schicht aus Gold aufweist.

Bevorzugterweise die Löt-Preform ringförmig oder scheibenförmig ausgebildet ist.

Insbesondere ist die Löt-Preform aus Gold und Zinn gebildet. Vorzugweise ist das Fenster zylinderförmig und/oder als Planglas ausgebildet.

In einer Ausgestaltung ist das Fenster aus Saphirglas hergestellt.

Ferner zeichnet sich eine Weiterbildung dadurch aus, dass die metallische Beschichtung auf der Planfläche des Fensters ringförmig ausgebildet ist und/oder eine Ringbreite zwischen 50 µm und 1000 µm, insbesondere zwischen 100 µm und 500 µm, aufweist.

Weitere Merkmale bevorzugter Ausführungsformen der Erfindung werden aus der Beschreibung von Ausführungsformen zusammen mit den Ansprüchen und den beigefügten Zeichnungen ersichtlich.

Im Rahmen der folgenden Beschreibung sind Merkmale, die mit "insbesondere" oder "vorzugsweise" gekennzeichnet sind, als fakultative Merkmale zu verstehen.

Es zeigen:
- Fig. 1: eine schematische und vereinfachte Seitenansicht eines chirurgischen Instruments,
- Fig. 2: eine Detailansicht einer Okulareinrichtung in einem schematischen und vereinfachten Längsschnitt und
- Fig. 3: eine perspektivische Ansicht einer Fensterscheibe für eine Fensteröffnung in einer Faserrohrspitze eines Endoskops,
- Fig. 3a, 3b: schematische Ansichten einer Anordnung einer Fensterscheibe in einer Faserrohrspitze eines Endoskops vor dem Verlöten (Fig. 3a) und im verlöteten Zustand (Fig. 3b),
- Fig. 4: schematisch eine Ansicht einer Okulareinrichtung eines chirurgischen Instruments in einem vereinfachten Längsschnitt.

In den Zeichnungen sind jeweils gleiche oder gleichartige Elemente und/oder Teile mit denselben Bezugsziffern versehen, so dass von einer erneuten Vorstellung jeweils abgesehen wird.

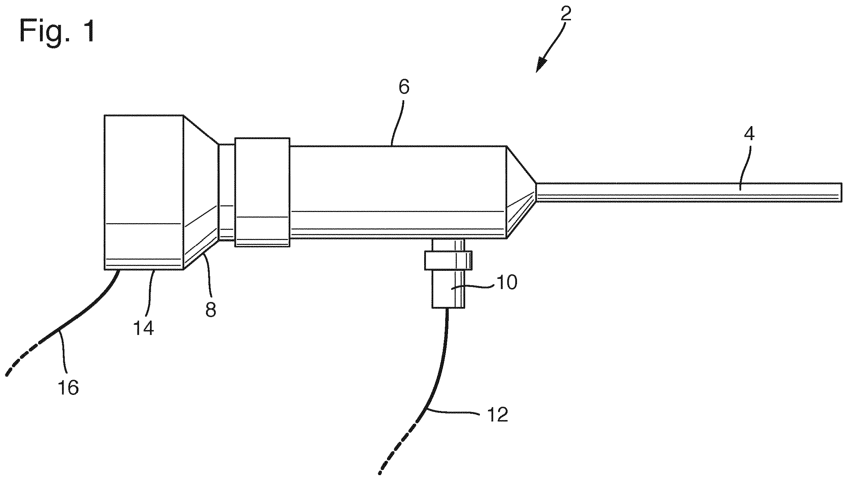

Fig. 1 zeigt in schematischer und vereinfachter Seitenansicht ein chirurgisches Instrument 2, beispielhaft ein Endoskop. An seinem distalen Ende umfasst dieses einen rohrförmigen Endoskopschaft 4 mit einer Optik, welche es erlaubt, einen Operations- oder Untersuchungsbereich, welcher distal vor dem freien Ende des Endoskopschafts 4 liegt, zu beobachten. Der Endoskopschaft 4 mündet in ein Gehäuse 6, das am proximalen Ende einen Okulartrichter 8 aufweist. Das Gehäuse 6 dient der Handhabung des chirurgischen Instruments 2. Seitlich an dem Gehäuse 6 befindet sich eine Lichtquelle 10, beispielsweise eine LED-Lichtquelle. Diese ist über ein Anschlusskabel 12 mit einer geeigneten Stromversorgung verbunden.

Am Okulartrichter 8 ist, schematisch dargestellt, ein Kamerakopf 14 mit einem nicht dargestellten Okularadapter angeordnet. Der Kamerakopf 14 erfasst das aus dem Okular des chirurgischen Instruments 2 austretende Licht mit einer eigenen Optik und bildet dieses auf einem optischen Flächensensor, beispielweise einem CCD- oder CMOS-Chip ab. Mittels eines Anschlusses 16 wird der Kamerakopf 14 mit Strom versorgt. Ferner ist es über den Anschluss 16 möglich, Bildsignale von dem Flächensensor des Kamerakopfs 14 einer externen Auswerteeinheit zu übermitteln und Steuersignale an den Kamerakopf 14 zu übertragen.

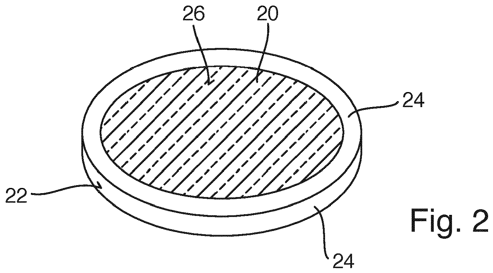

In Fig. 2 ist schematisch in einer perspektivischen Ansicht eine Fensterscheibe 20 dargestellt, die als Fenster beispielsweise in eine Fensteröffnung bzw. Fensteraufnahme an einer distalen Faserrohrspitze eines Faserrohrs 30 eines Endoskops oder in eine proximale Fensterfassung (Fensteraufnahme) einer Okulareinrichtung eingesetzt wird. Die Fensterscheibe 20 ist hierbei zylindrisch bzw. zylinderförmig mit einer zylindrischen Umfangsfläche 22 und zwei Planflächen 26 ausgebildet und weist an ihrer zylindrischen Umfangsfläche 22 eine Goldbeschichtung 24 auf. Aufgrund der perspektivischen Darstellung ist nur eine, d. h. die oberseitige, Planfläche 26 der Fensterscheibe 20 zu sehen.

Die Fensterscheibe 20 ist vorzugsweise als Planglas ausgebildet und weist auf der oberen bzw. sichtbaren Planfläche 26 im Randbereich eine ringförmige Goldbeschichtung 24 auf. Bei der Fensterscheibe 20 weist nur eine der beiden Planflächen 26 die ringförmige Goldbeschichtung 24 am äußeren Rand der Planfläche 26 auf. Vorzugsweise ist die Fensterscheibe 20 aus Saphirglas hergestellt, wobei die Fensterscheibe 20 in dem nicht-beschichteten inneren Bereich, der von der Goldbeschichtung 24 eingerahmt bzw. umgeben ist, durchsichtig bzw. für sichtbares Licht durchlässig ist.

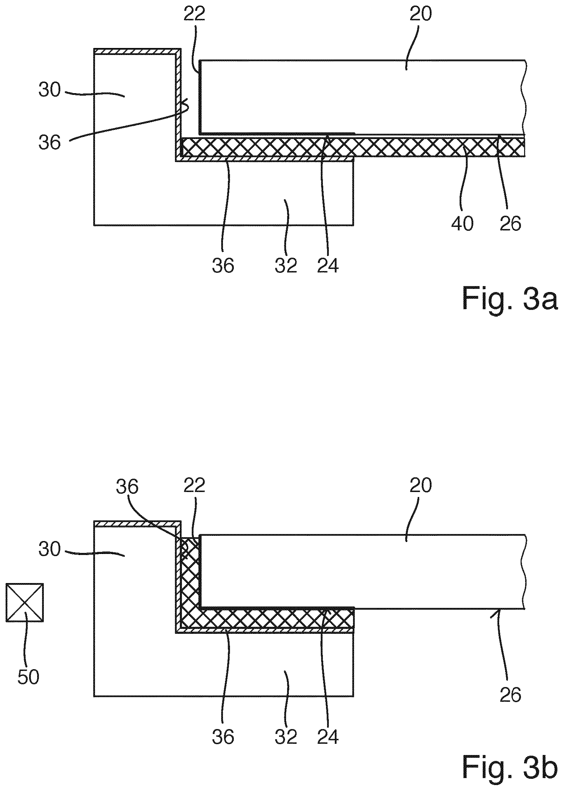

Die Fensterscheibe 20 wird in eine entsprechende Fensteraufnahme einer Faserrohrspitze (vgl. Fig. 3a, 3b) eingesetzt und durch Löten an der Faserrohrspitze mit dem Faserrohr verbunden, so dass die Fensterscheibe 20 in der Fensteraufnahme der Faserrohrspitze eingelötet ist.

Es ist vorgesehen, dass die Beschichtungen auf der Umfangsfläche 22 und auf der Planfläche der Fensterscheibe 20 auch mehrere Schichten, vorzugsweise metallische Schichten, aufweisen können, wobei die äußere Schicht der mehrlagigen Schichten hierbei aus Gold besteht oder Gold enthält.

Die ringförmige Goldbeschichtung 24 auf der Oberseite bzw. der Planfläche der Fensterscheibe 20 weist hierbei eine Breite zwischen 100 µm bis 500 µm auf. Insbesondere beträgt die Breite des Rings der Goldbeschichtung 24 auf der Planfläche 26 ungefähr 300 µm.

In Fig. 3a ist schematisch und im Ausschnitt die Anordnung der Fensterscheibe 20 an einer Faserrohrspitze eines Faserrohrs 30 am distalen Ende im Querschnitt gezeigt. Die Faserrohrspitze ist im Inneren des Endoskopschafts 4 (vgl. Fig. 1) am distalen Ende des Endoskopschafts 4 angeordnet. Im Inneren des Faserrohrs 30 befinden sich typischerweise am distalen Ende des Endoskopschafts 4 hier nicht dargestellte optische Komponenten des Endoskops.

Am distalen Ende des Faserrohrs 30 ist dieses nach innen mit einem Ringkörper 32 versehen, so dass auf der Innenseite des Ringkörpers 32 eine Auflagefläche für den Randbereich der Fensterscheibe 20 ausgebildet ist. In Fig. 3a ist schematisch die Anordnung der Fensterscheibe 20 an der Faserrohrspitze des Faserrohrs 30 vor dem Einlöten der Fensterscheibe 20 gezeigt.

Das Faserrohr 30 ist an den der Fensterscheibe 20 zugewandten Kontaktflächen an der Innenseite mit einer Goldbeschichtung 36 versehen. Ferner ist die Auflagefläche des Ringkörpers 32, die der Fensterscheibe 20 zugewandt ist, ebenfalls mit der Goldbeschichtung 36 versehen. Zum Einlöten der Fensterscheibe 20 in die Fassung bzw. Fensterfassung der Faserrohrspitze ist zwischen der Unterseite bzw. der Planfläche 26 der Fensterscheibe 20, die mit der Goldbeschichtung 24 versehen ist, und der Auflagefläche des Ringkörpers 32, die mit der Goldbeschichtung 36 ausgebildet ist, eine Löt-Preform 40 angeordnet. Die Löt-Preform 40 ist hierbei vorzugsweise ringförmig oder scheibenförmig ausgebildet und zwischen der Fensterscheibe 20 und dem Ringkörper 32 angeordnet. Vorzugsweise besteht die Löt-Preform 40 aus Gold und Zinn, z. B. aus 80 % Gold und 20% Zinn.

Um die Fensterscheibe 20 in die Fassung der Faserrohrspitze einzulöten, wird die Löt-Preform 40 mittels einer schematisch dargestellten induktiven Heizeinrichtung 50 erwärmt, wodurch das Lotmaterial fließfähig wird und dadurch das Lotmaterial der Löt-Preform 40 aufgrund von Kapillarkräften zwischen der Fensterscheibe 20 und dem Kantenbereich zwischen der Fensterscheibe 20 und dem Faserrohr 30 in den Zwischenraum zwischen dem Faserrohr 30 und der Fensterscheibe 20 fließt, wodurch der Kantenbereich bzw. der Zwischenraum zwischen dem Fenster 20 und dem Faserrohr 30 mit Lotmaterial ausgefüllt wird und dadurch die Fensterscheibe 20 in der Faserrohrspitze verlötet wird. Dies ist in Fig. 3 in einer schematischen Darstellung gezeigt.

In Fig. 3 ist ebenfalls schematisch eine induktive Heizvorrichtung 50 dargestellt, die zum Erwärmen der Löt-Preform 40 die Faserrohrspitze umgibt. Durch Bestromung der außerhalb des Faserrohrs 30 angeordneten induktiven Heizvorrichtung 50 wird die Löt-Preform 40 erwärmt.

Durch das gleichzeitige Erwärmen der kompletten Löt-Preform 40 mittels der induktiven Heizvorrichtung 50 wird erreicht, dass aufgrund der Kapillarkräfte zwischen der Fensterscheibe 20 und der Faserrohrspitze die Fensterscheibe 20 selbstzentrierend in der Öffnung des Faserrohrs 30 angeordnet und verlötet wird.

Vorzugsweise ist die Löt-Preform 40 scheibenförmig oder ringförmig ausgebildet. Insbesondere ist das Faserrohr 30 aus Metall hergestellt.

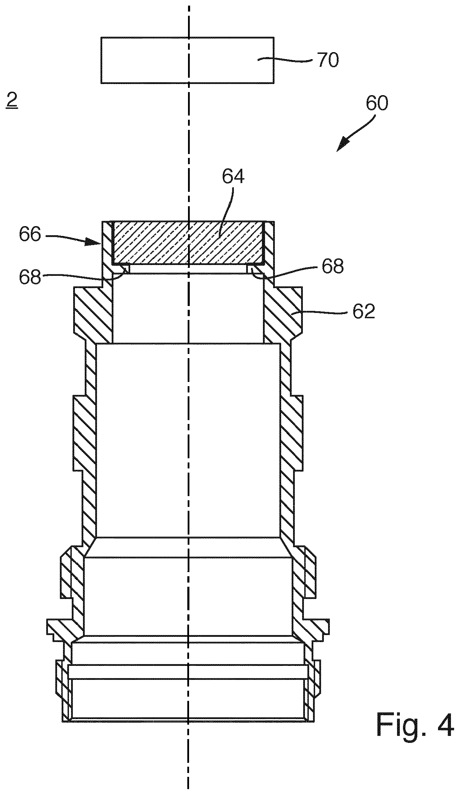

In Fig. 4 ist schematisch eine Okulareinrichtung 60 für ein chirurgisches Instrument 2 in einem schematischen und vereinfachten Längsschnitt dargestellt. Die Okulareinrichtung 60 ist am proximalen Ende eines chirurgischen Instruments 2, beispielsweise eines Endoskops, vorgesehen. Die Okulareinrichtung 60 weist hierbei eine Okularfensterfassung 62 auf, in der eine optische Baugruppe aufgenommen wird oder ist. Bei der optischen Baugruppe handelt es sich hierbei um eine oder mehrere Linsen oder Linsengruppen, Prismen, Filter oder dergleichen, welche ein optisches System bilden.

In Fig. 4 ist schematisch die optische Achse des optischen Systems in strichpunktierter Linie dargestellt. Die Okulareinrichtung 60 befindet sich in einem Innenraum des chirurgischen Instruments 2, beispielsweise in einem von dem Gehäuse 6 des in Fig. 1 dargestellten Endoskops umschlossenen Innenraum. Insbesondere bildet die Okularfensterfassung 62 die Trennung zwischen dem hermetischen Innenraum und dem nicht hermetischen Außenraum.

An der Lichtaustrittseite weist die Okularfensterfassung 62 ein Okularfenster 64 auf. Das Okularfenster 64 ist hierbei in einer Fensterfassung 66 aufgenommen und mit dieser verlötet. Die distale Fensterfassung 66 umgibt hierbei in Umfangsrichtung des Okularfensters 64 das Okularfenster 64, wobei das Okularfenster 64 zum Innenraum des chirurgischen Instruments 2 in Kontakt mit einer Halteschulter 68 der Okularfensterfassung 62 ist.

Das Okularfenster 64 ist in ähnlicher Weise wie die in Fig. 2 dargestellte Fensterscheibe 20 an der äußeren Umfangsfläche mit einer Goldbeschichtung und zusätzlich auf der Planebene bzw. Planfläche versehen, die dem Innenraum des Endoskops zugewandt ist, im äußeren Umfangsbereich mit einer ringförmigen Goldbeschichtung versehen.

Die Fensterfassung 66 ist an den Innenseiten, die der Umfangsfläche des zylindrischen Okularfensters 64 zugewandt ist, mit einer Goldbeschichtung 24 versehen. Auch die Auflagefläche der Halteschulter 68, die der ringförmigen Beschichtung des Okularfensters 64 zugewandt ist, ist ebenfalls mit einer Goldbeschichtung 24 versehen. Um unter Verwendung einer (hier nicht gezeigten) Löt-Preform zwischen der Fensterfassung 66 und dem Okularfenster 64 diese miteinander zu verlöten, ist ein Laser, zum Beispiel ein Diodenlaser, an der Okulareinrichtung 60 angeordnet, um die Löt-Preform 40 (hier nicht dargestellt) zu erwärmen. Durch die Erwärmung der Löt-Preform wird der Zwischenraum zwischen der Okularfassung 66 und dem Okularfenster 64 ausgefüllt, wodurch das Okularfenster 64 in die Okularfassung 66 eingelötet wird.

Vorzugsweise ist die Okularfensterfassung 62 aus Stahl oder aus einem Kunststoff, wie zum Beispiel PEEK, hergestellt.

### Bezugszeichenliste

- 2: chirurgisches Instrument
- 4: Endoskopschaft
- 6: Gehäuse
- 8: Okulartrichter
- 10: Lichtquelle
- 12: Anschlusskabel
- 14: Kamerakopf
- 16: Anschluss
- 20: Fensterscheibe
- 22: Umfangsfläche
- 24: Goldbeschichtung
- 26: Planfläche
- 30: Faserrohr
- 32: Ringkörper
- 36: Goldbeschichtung
- 40: Löt-Preform
- 50: induktive Heizvorrichtung
- 60: Okulareinrichtung
- 62: Okularfensterfassung
- 64: Okularfenster
- 66: Fensterfassung
- 68: Schulter
- 70: Lasereinrichtung

## Patentansprüche

1. Verfahren zum Herstellen eines Endoskops (2) mit den folgenden Verfahrensschritten:
- Bereitstellen eines eine seitliche Umfangsfläche (22) und zwei Planflächen (26) aufweisenden Fensters (20, 64), wobei das Fenster (20, 64) an der seitlichen Umfangsfläche (22) und/oder im Randbereich einer Planfläche (26) des Fensters (20, 64) mit einer metallischen Beschichtung (24) versehen ist, und wobei eine oder die, vorzugweise mit der metallischen Beschichtung (24) versehene, Planfläche (26) des Fensters (20, 64) mit einer Löt-Preform (40) versehen ist oder wird,
- Einsetzen des mit der Löt-Preform (40) versehenen Fensters (20, 64) in eine Fensteraufnahme eines Endoskopschafts des Endoskops, wobei insbesondere die mit der Löt-Preform (40) versehene Planfläche (26) des Fensters (20, 64) dem Innenraum des Endoskopschafts (4) zugewandt oder vom Innenraum des Endoskopschafts (4) abgewandt ist,
- Erwärmen, vorzugsweise gleichzeitiges Erwärmen, der Löt-Preform (40) an mehreren Stellen oder, vorzugsweise gleichzeitiges, Erwärmen der gesamten Löt-Preform (40) mittels einer Heizvorrichtung (50, 70), so dass das fließfähige Lotmaterial der Löt-Preform (40) zwischen dem Fenster (20, 64) und der Fensteraufnahme verteilt wird und nach Abkühlung des Lotmaterials ein Lot zwischen dem Fenster (20, 64) und der Fensteraufnahme angeordnet ist.

2. Verfahren nach Anspruch 1, **dadurch gekennzeichnet, dass** die metallische Beschichtung (24) des Fensters (20, 64) aus Gold besteht oder eine äußere Schicht aus Gold aufweist.

3. Verfahren nach Anspruch 1 oder 2, **dadurch gekennzeichnet, dass** die Fensteraufnahme Flächen, die der seitlichen Umfangsfläche (22) des Fensters (20, 64) und der Planfläche (26) des Fensters (20, 64) zugewandt sind, aufweist, wobei wenigstens eine Fläche oder beide Flächen eine Goldbeschichtung oder eine äußere Goldschicht aufweisen.

4. Verfahren nach einem der Ansprüche 1 bis 3, **dadurch gekennzeichnet, dass** die Löt-Preform (40) ringförmig oder scheibenförmig ausgebildet ist und/oder dass die Löt-Preform (40) aus Gold und Zinn gebildet ist.

5. Verfahren nach einem der Ansprüche 1 bis 4, **dadurch gekennzeichnet, dass** die Heizvorrichtung als induktive Heizvorrichtung (50, 70) oder als Lasereinrichtung ausgebildet ist.

6. Verfahren nach einem der Ansprüche 1 bis 5, **dadurch gekennzeichnet, dass** das Fenster (20, 64) zylinderförmig und/oder als, vorzugsweise scheibenförmiges, Planglas ausgebildet ist und/oder das Fenster (20, 64) aus Saphirglas hergestellt ist.

7. Verfahren nach einem der Ansprüche 1 bis 6, **dadurch gekennzeichnet, dass** die Fensteraufnahme aus Stahl oder aus Kunststoff, insbesondere PEEK (Polyetheretherketon), hergestellt ist.

8. Verfahren nach einem der Ansprüche 1 bis 7, **dadurch gekennzeichnet, dass** die Fensteraufnahme am distalen Ende des Endoskopschafts (4) oder am proximalen Ende des Endoskopschafts (4) ausgebildet ist.

9. Verfahren nach einem der Ansprüche 1 bis 8, **dadurch gekennzeichnet, dass** die metallische Beschichtung (24) auf der Planfläche (26) des Fensters (20, 64) ringförmig ausgebildet ist und/oder eine Ringbreite zwischen 50 µm und 1000 µm, insbesondere zwischen 100 µm und 500 µm, aufweist.

10. Verwendung eines Fensters (20, 64) und einer Löt-Preform (40) zur Herstellung eines einen Endoskopschafts (4) aufweisenden Endoskops, wobei das Fenster (20, 64) eine seitliche Umfangsfläche (22) und zwei Planflächen (26) aufweist, wobei das Fenster (20, 64) an der seitlichen Umfangsfläche (22) und/oder im Randbereich einer Planfläche (26) des Fensters (20, 64) mit einer metallischen Beschichtung (24) versehen ist, und wobei eine oder die, vorzugweise mit der metallischen Beschichtung (24) versehene, Planfläche (26) des Fensters (20, 64) mit einer Löt-Preform (40) versehen ist oder wird, wobei das Fenster (20, 64) in eine Fensteraufnahme des Endoskopschafts (4) einsetzbar ist oder eingesetzt wird, wobei die mit der Löt-Preform (40) versehene Planfläche (26) des Fensters (20, 64) dem Innenraum des Endoskopschafts (4) zugewandt oder vom Innenraum des Endoskopschafts (4) abgewandt ist.

11. Verwendung nach Anspruch 10, **dadurch gekennzeichnet, dass** durch gleichzeitiges Erwärmen der Löt-Preform (40) an mehreren Stellen oder durch gleichzeitigen Erwärmen der gesamten Löt-Preform (40) mittels einer Heizvorrichtung (50, 70) das fließfähige Lotmaterial der Löt-Preform zwischen dem Fenster (20, 64) und der Fensteraufnahme verteilbar oder verteilt ist und nach Abkühlung des Lotmaterials ein Lot zwischen dem Fenster (20, 64) und der Fensteraufnahme angeordnet ist.

12. Verwendung nach Anspruch 10 oder 11, **dadurch gekennzeichnet, dass** die metallische Beschichtung (24) des Fensters (20, 64) aus Gold besteht oder eine äußere Schicht aus Gold aufweist.

13. Verwendung nach einem der Ansprüche 10 bis 12, **dadurch gekennzeichnet, dass** die Löt-Preform (40) ringförmig oder scheibenförmig ausgebildet ist und/oder dass die Löt-Preform (40) aus Gold und Zinn gebildet ist.

14. Verwendung nach einem der Ansprüche 10 bis 13, **dadurch gekennzeichnet, dass** das Fenster (20, 64) zylinderförmig und/oder als Planglas ausgebildet ist und/oder dass das Fenster (20, 64) aus Saphirglas hergestellt ist.

15. Verwendung nach einem der Ansprüche 10 bis 14, **dadurch gekennzeichnet, dass** die metallische Beschichtung (24) auf der Planfläche (26) des Fensters (20, 64) ringförmig ausgebildet ist und/oder eine Ringbreite zwischen 50 µm und 1000 µm, insbesondere zwischen 100 µm und 500 µm, aufweist.

## Claims

1. A method for the manufacturing an endoscope (2) having the following method steps:
- Providing a window (20, 64) having one lateral peripheral surface (22) and two flat surfaces (26), wherein the window (20, 64) is provided with a metal coating (24) on the lateral peripheral surface (22) and/or in the edge region of a flat surface (26) of the window (20, 64), and wherein a or the flat surface (26) of the window (20, 64), preferably provided with the metal coating (24), is or will be provided with a solder preform (40),
- Inserting the window (20, 64) provided with the solder preform (40) into a window seat of an endoscope shaft of the endoscope, wherein in particular the flat surface (26) of the window (20, 64) provided with the solder preform (40) faces the interior space of the endoscope shaft (4) or faces away from the interior space of the endoscope shaft (4),
- Heating, preferably simultaneously heating, the solder preform (40) at several points, or, preferably simultaneously, heating the entire solder preform (40) with a heating device (50, 70) so that the flowable solder material of the solder preform (40) is distributed between the window (20, 64) and the window seat and, after the solder material cools, solder is arranged between the window (20, 64) and the window seat.

2. The method according to claim 1, **characterized in that** the metal coating (24) of the window (20, 64) consists of gold or has an outer layer of gold.

3. The method according to claim 1 or 2, **characterized in that** the window seat has surfaces which face the lateral peripheral surface (22) of the window (20, 64) and the flat surface (26) of the window (20, 64), wherein at least one surface, or both surfaces, have a gold coating or an outer gold layer.

4. The method according to one of the claims 1 to 3, **characterized in that** the solder preform (40) is designed annular or disk-shaped and/or that the solder preform (40) is made of gold and tin.

5. The method according to one of claims 1 to 4, **characterized in that** the heating device is designed as an inductive heating device (50, 70) or as a laser device.

6. The method according to one of claims 1 to 5, **characterized in that** the window (20, 64) is designed cylindrical and/or as a, preferably disk-shaped flat, glass and/or the window (20, 64) is produced from sapphire glass.

7. The method according to one of claims 1 to 6, **characterized in that** the window seat is produced from steel or plastic, in particular PEEK (polyetheretherketone).

8. The method according to one of claims 1 to 7, **characterized in that** the window seat is formed on the distal end of the endoscope shaft (4) or on the proximal end of the endoscope shaft (4).

9. The method according to one of claims 1 to 8, **characterized in that** the metal coating (24) is formed on the flat surface (26) of the window (20, 64) in a ring-shape and/or has a ring width between 50 µm and 1000 µm, in particular between 100 µm and 500 µm.

10. The use of a window (20, 64) and a solder preform (40) for manufacturing an endoscope having an endoscope shaft (4), wherein the window (20, 64) has one lateral peripheral surface (22) and two flat surfaces (26), wherein the window (20, 64) is provided with a metal coating (24) on the lateral peripheral surface (22) and/or in the edge region of a flat surface (26) of the window (20, 64), and wherein a or the flat surface (26) of the window (20, 64) preferably provided with the metal coating (24) is or will be provided with a solder preform (40), wherein the window (20, 64) is insertable or will be inserted in a window seat of the endoscope shaft (4), wherein the flat surface (26) of the window (20, 64) provided with the solder preform (40) faces the interior space of the endoscope shaft (4), or faces away from the interior space of the endoscope shaft (4).

11. The use according to claim 10, **characterized in that** by simultaneously heating the solder preform (40) at several points, or by simultaneously heating the entire solder preform (40) with a heating device (50, 70), the flowable solder material of the solder preform can be or is distributed between the window (20, 64) and the window seat and, after the solder material cools, solder is arranged between the window (20, 64) and the window seat.

12. The use according to claim 10 or 11, **characterized in that** the metal coating (24) of the window (20, 64) consists of gold or has an outer layer of gold.

13. The use according to one of the claims 10 to 12, **characterized in that** the solder preform (40) is designed annular or disk-shaped and/or the solder preform (40) is made of gold and tin.

14. The use according to one of claims 10 to 13, **characterized in that** the window (20, 64) is designed cylindrical and/or as a, preferably disk-shaped, flat glass and/or the window (20, 64) is produced from sapphire glass.

15. The use according to one of claims 10 to 14, **characterized in that** the metal coating (24) is formed on the flat surface (26) of the window (20, 64) in a ring shape and/or has a ring width between 50 µm and 1000 µm, in particular between 100 µm and 500 µm.

## Revendications

1. Procédé de fabrication d'un endoscope (2) comprenant les étapes de procédé suivantes:
- Préparation d'une fenêtre (20, 64) présentant une surface périphérique latérale (22) et deux surfaces planes (26), la fenêtre (20, 64) étant pourvue d'un revêtement métallique (24) sur la surface périphérique latérale (22) et/ou dans la zone de bordure d'une surface plane (26) de la fenêtre (20, 64), et une surface plane (26) ou la surface plane (26) de la fenêtre (20, 64), de préférence pourvue du revêtement métallique (24), étant pourvue ou étant apte à être pourvue d'une préforme à braser (40),
- Insertion de la fenêtre (20, 64) pourvue de la préforme à braser (40) dans un logement de fenêtre d'une tige d'endoscope de l'endoscope, la surface plane (26) de la fenêtre (20, 64) pourvue de la préforme à braser (40) étant notamment tournée vers l'intérieur de la tige d'endoscope (4) ou tournée à l'opposé de l'intérieur de la tige d'endoscope (4),
- Chauffage, de préférence simultanément, de la préforme à braser (40) en plusieurs endroits ou, de préférence simultanément, chauffage de l'ensemble de la préforme à braser (40) au moyen d'un dispositif de chauffage (50, 70), de sorte que le matériau de brasage fluide de la préforme à braser (40) est réparti entre la fenêtre (20, 64) et le logement de fenêtre et, après refroidissement du matériau de brasage, une brasure est agencée entre la fenêtre (20, 64) et le logement de fenêtre.

2. Procédé selon la revendication 1, **caractérisé en ce que** le revêtement métallique (24) de la fenêtre (20, 64) est en or ou présente une couche externe en or.

3. Procédé selon la revendication 1 ou la revendication 2, **caractérisé en ce que** le logement de fenêtre comprend des surfaces tournées vers la surface périphérique latérale (22) de la fenêtre (20, 64) et à la surface plane (26) de la fenêtre (20, 64), au moins une surface ou les deux surfaces comprenant un revêtement en or ou une couche extérieure en or.

4. Procédé selon l'une des revendications 1 à 3, **caractérisé en ce que** la préforme à braser (40) est en forme d'anneau ou de disque et/ou **en ce que** la préforme à braser (40) est formée d'or et d'étain.

5. Procédé selon l'une des revendications 1 à 4, **caractérisé en ce que** le dispositif de chauffage est sous la forme d'un dispositif de chauffage inductif (50, 70) ou d'un dispositif laser.

6. Procédé selon l'une des revendications 1 à 5, **caractérisé en ce que** la fenêtre (20, 64) est de forme cylindrique et/ou en verre plan, de préférence en forme de disque, et/ou la fenêtre (20, 64) est fabriquée en verre saphir.

7. Procédé selon l'une des revendications 1 à 6, **caractérisé en ce que** le logement de la fenêtre est fabriqué en acier ou en matière plastique, notamment en PEEK (polyétheréthercétone).

8. Procédé selon l'une des revendications 1 à 7, **caractérisé en ce que** le logement de fenêtre est formé à l'extrémité distale de la tige d'endoscope (4) ou à l'extrémité proximale de la tige d'endoscope (4).

9. Procédé selon l'une des revendications 1 à 8, **caractérisé en ce que** le revêtement métallique (24) est en forme d'anneau sur la surface plane (26) de la fenêtre (20, 64) et/ou présente une largeur annulaire comprise entre 50 µm et 1000 µm, notamment entre 100 µm et 500 µm.

10. Utilisation d'une fenêtre (20, 64) et d'une préforme à braser (40) pour la fabrication d'un endoscope présentant une tige d'endoscope (4), la fenêtre (20, 64) présentant une surface périphérique latérale (22) et deux surfaces planes (26), la fenêtre (20, 64) étant pourvue d'un revêtement métallique (24) sur la surface périphérique latérale (22) et/ou dans la zone de bordure d'une surface plane (26) de la fenêtre (20, 64), et une surface plane (26) ou les surfaces planes (26) de la fenêtre (20, 64), de préférence munie du revêtement métallique (24), étant pourvues ou étant aptes à être pourvues d'un revêtement métallique (24), d'une préforme à braser (40), la fenêtre (20, 64) étant apte à être insérée ou étant insérée dans un logement de fenêtre de la tige d'endoscope (4), la surface plane (26) de la fenêtre (20, 64) munie de la préforme à braser (40) étant orientée vers l'espace intérieur de la tige d'endoscope (4) ou étant orientée à l'opposé de l'espace intérieur de la tige d'endoscope (4).

11. Utilisation selon la revendication 10, **caractérisée en ce que**, par chauffage simultané de la préforme à braser (40) en plusieurs endroits ou par chauffage simultané de l'ensemble de la préforme à braser (40) au moyen d'un dispositif de chauffage (50, 70), le matériau de brasage fluide de la préforme à braser est apte à être réparti ou est réparti entre la fenêtre (20, 64) et le logement de fenêtre et, après refroidissement du matériau de brasage, une brasure est agencée entre la fenêtre (20, 64) et le logement de fenêtre.

12. Utilisation selon la revendication 10 ou la revendication 11, **caractérisée en ce que** le revêtement métallique (24) de la fenêtre (20, 64) est en or ou comporte une couche externe en or.

13. Utilisation selon l'une des revendications 10 à 12, **caractérisée en ce que** la préforme à braser (40) est selon une forme d'anneau ou de disque et/ou **en ce que** la préforme à braser (40) est formée d'or et d'étain.

14. Utilisation selon l'une des revendications 10 à 13, **caractérisée en ce que** la fenêtre (20, 64) est de forme cylindrique et/ou est en verre plan et/ou **en ce que** la fenêtre (20, 64) est fabriquée en verre saphir.

15. Utilisation selon l'une des revendications 10 à 14, **caractérisée en ce que** le revêtement métallique (24) est annulaire sur la face plane (26) de la fenêtre (20, 64) et/ou présente une largeur annulaire comprise entre 50 µm et 1000 µm, notamment entre 100 µm et 500 µm.
